# EUROPEAN PATENT APPLICATION

(11) **EP 4 378 533 A1**
(43) Date of publication of application: **05.06.2024**
(21) Application number: 22210358.2
(22) Date of filing: 29.11.2022
(51) Int. Cl.: A61P 37/00, C12Q 1/6876

(54) **PATIENT SELECTION AND THERAPY MONITORING FOR AUTOIMMUNE DISORDERS**

(71) Applicant: Fraunhofer-Gesellschaft zur Förderung der angewandten Forschung e.V., 80686 München (DE)
(72) Inventor: BURKHARDT, Harald, 60590 Frankfurt am Main (DE); HOLMDAHL, Rikard, 22457 Lund (SE); DO, Nhu-Nguyen, 60590 Frankfurt am Main (DE); EDINGER-JAKOBI, Katja, 60590 Frankfurt am Main (DE); TURGAY, Ninorta, 60590 Frankfurt am Main (DE)
(74) Representative: Kuttenkeuler, David

(57) **Abstract**

The invention pertains to V-type immunoglobulin domain-containing suppressor of T-cell activation (VISTA) protein or mRNA as a diagnostic marker for the selection of patients suffering from autoimmune disorders for therapy, and as a means for monitoring therapy success of autoimmune disorders. The invention pertains to methods of patient selection and therapy monitoring, combined diagnostic and therapeutic applications involving determination of VISTA proteins and/or mRNA in patient samples, as well as diagnostic kits suitable for use in a method of the invention.

## Description

### FIELD OF THE INVENTION

The invention pertains to V-type immunoglobulin domain-containing suppressor of T-cell activation (VISTA) protein or mRNA as a diagnostic marker for the selection of patients suffering from autoimmune disorders for therapy, and as a means for monitoring therapy success of autoimmune disorders. The invention pertains to methods of patient selection and therapy monitoring, combined diagnostic and therapeutic applications involving determination of VISTA proteins and/or mRNA in patient samples, as well as diagnostic kits suitable for use in a method of the invention.

### DESCRIPTION

In autoimmune diseases the patient's immune system has lost the ability to discriminate between body-own ("self') and foreign proteins. In consequence, antibodies and/or T cell responses are generated that recognize "self-determinants" e.g. proteins, carbohydrates, nucleic acids and form immune complexes which continuously activate the immune system because the "self'-structures are permanently produced and recognized as foreign. This chronic condition can persist for years leading in the end to severe tissue and organ damage and possibly even to the death of the patient. There are many different autoimmune disorders which affect the body in various ways. For example, the brain is affected in individuals with multiple sclerosis, the gut is affected in individuals having Crohn's disease, and the synovium, bone and cartilage of various joints are affected in individuals suffering from rheumatoid arthritis. As autoimmune disorders progress destruction of one or more types of body tissues, abnormal growth of an organ, or changes in organ function may result. The autoimmune disorder may affect a single organ or tissue type or may affect multiple organs and tissues. Organs and tissues commonly affected by autoimmune disorders include red blood cells, blood vessels, connective tissues, endocrine glands (e.g., the thyroid or pancreas), muscles, joints, and the skin.

Examples of inflammatory and/or autoimmune disorders include, but are not limited to, primary immune thrombocytopenia (ITP), systemic lupus erythematosus (SLE), rheumatoid arthritis (RA), autoimmune haemolytic anaemia (AIHA), type I diabetes, Pemphigus vulgaris, Hashimoto's thyroiditis, autoimmune inner ear disease myasthenia gravis, pernicious anemia, Addison's disease, dermatomyositis, Sjögren's syndrome, dermatomyositis, multiple sclerosis, Reiter's syndrome, Graves disease, autoimmune hepatitis, familial adenomatous polyposis and ulcerative colitis.

V-set immunoregulatory receptor (VSIR), initially described and designated as "V-domain Ig suppressor of T cell activation" (VISTA) by Wang et al (2011; J Exp Med 208:777), is an immunoglobulin (Ig) superfamily ligand that negatively regulates T cell responses. VISTA is primarily expressed on hematopoietic cells, and VISTA expression is highly regulated on myeloid antigen-presenting cells (APCs) and T cells. Wang et al described that a soluble VISTA-Ig fusion protein or VISTA expression on APCs inhibited T cell proliferation and cytokine production *in vitro,* and that a VISTA-specific monoclonal antibody interfered with VISTAinduced suppression of T cell responses by VISTA-expressing APCs in vitro. These findings showed that VISTA had functional activities that were non-redundant with other Ig superfamily members, and Wang et al postulated further that VISTA might play a role in the development of autoimmunity and immune surveillance in cancer. VISTA is since known as a broad-spectrum negative checkpoint regulator for cancer immunotherapy (Lines et al, 2014; Cancer Immunol Res 2:510).

VISTA has been associated with acquired resistance to anti-PD-i therapy in metastatic melanoma patients (Kakavand et al, 2017; Modern Pathol 89, doi:10.1038/modpathol.2017.89; published online 4 Aug. 2017), and as a compensatory inhibitory pathway in prostate tumours after ipilimumab therapy (Gao et al, 2017; Nat Med 23:551). Furthermore, the immunecheckpoint protein VISTA has been described to critically regulate the IL-23/IL-17 inflammatory axis (Li et al, 2017; Sci Rep 7:1485).

New approaches are needed for the very early stages after clinical onset, but disease prevention is the ultimate goal. To improve treatment, the antigen-specific autoimmunity regulating the onset of joint inflammation must be addressed. Before and around clinical onset, autoimmune responses include citrullinated proteins in respective genetically predisposed individuals. In addition, an immune response to joint proteins, including the major joint protein, type II collagen (COL2) and to citrullinated COL2, appears. COL2 is of particular interest, as it is the major structural protein in cartilage as well as an autoantigenic component expressed in central lymphoid organs, the thymus and bone marrow. Indeed, immunization of COL2 with potent adjuvant induces the development of autoimmune arthritis in rodents (collagen-induced arthritis [CIA] model). Experimental arthritis is dependent on major histocompatibility complex region class II (MHCII) interaction; in the mouse, CIA is associated with the MHCII allele Aq and is strictly dependent on T-cell recognition of the galactosylated 259-273 COL2 epitope. Interestingly, T cells specific for the COL2₂₅₉₋₂₇₃ peptide occur in RA patients carrying the DRB1^{∗}0401 allele. COL2-reactive T cells are detectable in both healthy individuals and in RA patients and they predominantly recognize the immunodominant COL2₂₅₉₋₂₇₃ peptide when it is glycosylated at positions 264 and/or 270. The function of these frequently occurring COL2-reactive T cells is not clear, as these cells could have regulatory or pathogenic functions. However, humanized mice expressing human DRB1^{∗}0401 MHCII develop CIA mediated by the COL2₂₅₉₋₂₇₃-specificT cells.

WO 2021/028347 discloses HLA-DR/COL2 peptide complexes comprising a chondroitin-binding peptide sequence at the C-terminal end of the polypeptide comprising the HLA-DR alpha chain and/or the HLA-DR beta chain, wherein the COL2 peptide is fused to the N-terminus of the HLA-DR alpha chain or the HLA-DR beta chain by a linker peptide. The complexes are for use in treating chronic inflammatory disease, such as arthritis, in human patients.

International patent publication WO 2021/028350 shows in situ glycosylated MHC II/COL2 peptide complexes, i.e., complexes naturally glycosylated during recombinant protein expression in the host cell. WO 2021/028350 also relates to methods of producing glycosylated MHC II/COL2 peptide complexes in mammalian cells as well as the use of such posttranslationally modified, preferably glycosylated MHC/COL2 complexed in treating rheumatoid arthritis.

A variety of Co-inhibitory immune receptors have been characterized to be upregulated on natural as well as induced regulatory T cells. These include i.e. CTLA-4, PD-1, Lag-3, Tim-3, and TIGIT. The immunoregulatory function of these molecules is well characterized and their expression on CD4+ T cells is a commonly used as a marker of a regulatory T cell phenotype. These markers and additional molecules e.g. the transcription factor FOXP3, FR4 (folate receptor 4) and CD49b (a2 integrin chain), CD73 (ecto-5'-nucleotidase) and the secretion of interleukin-10 (review in 2) were tested in the development of a vaccine approach as an indicators of the pharmacodynamic effect on the CD4+ T cell population. The above-mentioned markers were proven suitable to characterize the lymphocyte phenotype induced by therapeutic vaccination of collagen-induced arthritis (CIA) with a murine homologue to the human MHCII/galCOL2 peptide complex was suggested to be similar to a Type 1 regulatory (TRi) cell. However, the respective *in vitro* stimulation of patient derived PBMCs with the respective human vaccine did not consistently upregulate the entire set of TRi markers. Accordingly, the set of already established prior art markers for induced regulatory cells alone will not allow for an immune monitoring of the innovative treatment of autoimmune disorders as suggested in the above summarized prior art.

Thus, it is an object of the invention to provide novel means to allow an effective selection of patients suffering from autoimmune disorders such as rheumatoid arthritis, as well monitoring of treatment success, which enables clinicians to effectively control therapy in patients.

### BRIEF DESCRIPTION OF THE INVENTION

The above problem in the prior art is solved in a general aspect of the invention by VISTA protein or RNA as a detectable biomarker indicative for treatment susceptibility and success. Generally, and by way of brief description, the main aspects of the present invention can be described as follows:
In **a first aspect,** the invention pertains to a method for an evaluation of a treatment of an autoimmune disorder in a subject who receives or received the treatment, the method comprising the steps of
   (a) Providing a biological test sample comprising T-cells of the subject;
   (b) Determining (i) the level of expression of a V-type immunoglobulin domain-containing suppressor of T-cell activation (VISTA) protein, or VISTA RNA, in the T-cells of the biological test sample; or (ii) determining the number or percentage of VISTA protein and/or VISTA RNA positive T-cells in the biological test sample;
   **(c)** Comparing the values as determined in step (b)(i) and/or (b)(ii) with a reference preferably wherein reference for (i) is a predetermined level of VISTA protein or VISTA RNA, or wherein the reference for (i) is a level of VISTA protein or VISTA RNA determined in T-cells of a control sample such as a biological sample comprising T-cells of the subject at a time before the treatment or at a time during treatment, but before obtaining the biological test-sample; and/or preferably wherein the reference for (ii) is a predetermined number/percentage of VISTA protein and/or VISTA RNA positive T-cells, or wherein the reference for (ii) is a number/percentage of VISTA protein and/or VISTA RNA positive T-cells of a control sample such as a biological control sample comprising T-cells of the subject obtained at a time before the treatment or obtained at a time during treatment, but at an earlier time than the biological test sample was obtained;
   wherein an increased level in (i) and/or an increased number/percentage in (ii) compared to the respective reference indicates treatment success.
In **a second aspect,** the invention pertains to a method for the evaluation of whether a subject is susceptible to a treatment of an autoimmune disorder in a subject who suffers from the autoimmune disorder and has not yet received the treatment, the method comprising the steps of
   **(a)** Providing a biological test sample comprising T-cells of the subject;
   **(b)** Determining (i) the level of expression of a V-type immunoglobulin domain-containing suppressor of T-cell activation (VISTA) protein, or VISTA RNA, in the T-cells of the biological test sample; or (ii) determining the number or percentage of VISTA protein and/or VISTA RNA positive T-cells in the biological test sample;
   **(c)** Comparing the values as determined in step (b)(i) and/or (b)(ii) with a reference;
      preferably wherein reference for (i) is a predetermined level of VISTA protein or VISTA RNA, or wherein the reference for (i) is a level of VISTA protein or VISTA RNA determined in T-cells of a control sample such as a biological sample comprising T-cells of the subject at a time before the treatment or at a time during treatment, but before obtaining the biological test-sample; and/or
      preferably wherein the reference for (ii) is a predetermined number/percentage of VISTA protein and/or VISTA RNA positive T-cells, or wherein the reference for (ii) is a number/percentage of VISTA protein and/or VISTA RNA positive T-cells of a control sample such as a biological control sample comprising T-cells of the subject obtained at a time before the treatment or obtained at a time during treatment, but at an earlier time than the biological test sample was obtained;
   wherein an increased level in (i) and/or an increased number/percentage in (ii) compared to the respective reference indicates that the subject is susceptible to the treatment.
In **a third aspect,** the invention pertains to a compound or composition for use in the treatment of an autoimmune disorder in a subject, wherein the compound and composition is immune-regulatory and negatively affects a T-cell mediated immune response in the subject when administered, and wherein the treatment further comprises obtaining a biological test sample of the subject at least once before, during and/or subsequent to the administration of the compound or composition, and wherein the subject is stratified as being successfully treated or being susceptible to the treatment by a method according to any one of claim 1 to 30 using the biological test sample of the subject.

### DETAILED DESCRIPTION OF THE INVENTION

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

In **a first aspect,** the invention pertains to a method for the evaluation of a treatment of an autoimmune disorder in a subject who receives or received the treatment, the method comprising the steps of
(a) Providing a biological test sample comprising T-cells of the subject;
(b) Determining (i) the level of expression of a V-type immunoglobulin domain-containing suppressor of T-cell activation (VISTA) protein, or VISTA RNA, in the T-cells of the biological test sample; or (ii) determining the number or percentage of VISTA protein and/or VISTA RNA positive T-cells in the biological test sample;
(c) Comparing the values as determined in step (b)(i) and/or (b)(ii) with a reference preferably wherein reference for (i) is a predetermined level of VISTA protein or VISTA RNA, or wherein the reference for (i) is a level of VISTA protein or VISTA RNA determined in T-cells of a control sample such as a biological sample comprising T-cells of the subject at a time before the treatment or at a time during treatment, but before obtaining the biological test-sample; and/or preferably wherein the reference for (ii) is a predetermined number/percentage of VISTA protein and/or VISTA RNA positive T-cells, or wherein the reference for (ii) is a number/percentage of VISTA protein and/or VISTA RNA positive T-cells of a control sample such as a biological control sample comprising T-cells of the subject obtained at a time before the treatment or obtained at a time during treatment, but at an earlier time than the biological test sample was obtained;
wherein an increased level in (i) and/or an increased number/percentage in (ii) compared to the respective reference indicates treatment success.

Therefore, the invention provides a use of VISTA expression (protein or RNA) on T lymphocytes as a quantitative biomarker for a treatment-induced regulatory phenotype of CD4+ T cells in patients with (auto)immune diseases i.e. rheumatoid arthritis (RA). The invention is based on the identification of VISTA as a biomarker whose upregulation indicates a regulatory phenotype of CD4+ T cells and can be used to measure the impact of a therapeutic intervention by T cell directed immunoregulatory agents i.e. protein complexes consisting of a MHC (major histocompatibility complex) II protein and a peptide attached to its binding groove for selective T cell receptor (TCR) mediated activation of a subset of T lymphocytes. This biomarker is useful for the prediction of responsiveness to treatment as well as a parameter of effectiveness in the immune-monitoring of initiated therapy.

Hence, in **a second aspect,** the invention pertains to a method for the evaluation of whether a subject is susceptible to a treatment of an autoimmune disorder in a subject who suffers from the autoimmune disorder and has not yet received the treatment, the method comprising the steps of
**(a)** Providing a biological test sample comprising T-cells of the subject;
**(b)** Determining (i) the level of expression of a V-type immunoglobulin domain-containing suppressor of T-cell activation (VISTA) protein, or VISTA RNA, in the T-cells of the biological test sample; or (ii) determining the number or percentage of VISTA protein and/or VISTA RNA positive T-cells in the biological test sample;
**(c)** Comparing the values as determined in step (b)(i) and/or (b)(ii) with a reference;
   preferably wherein reference for (i) is a predetermined level of VISTA protein or VISTA RNA, or wherein the reference for (i) is a level of VISTA protein or VISTA RNA determined in T-cells of a control sample such as a biological sample comprising T-cells of the subject at a time before the treatment or at a time during treatment, but before obtaining the biological test-sample; and/or
   preferably wherein the reference for (ii) is a predetermined number/percentage of VISTA protein and/or VISTA RNA positive T-cells, or wherein the reference for (ii) is a number/percentage of VISTA protein and/or VISTA RNA positive T-cells of a control sample such as a biological control sample comprising T-cells of the subject obtained at a time before the treatment or obtained at a time during treatment, but at an earlier time than the biological test sample was obtained;
wherein an increased level in (i) and/or an increased number/percentage in (ii) compared to the respective reference indicates that the subject is susceptible to the treatment.

Information on "V-domain Ig suppressor of T cell activation" (VISTA) by Wang et al (2011), is described above, and "V-set immunoregulatory receptor"-or "VSIR" (or "VISTA")-as a protein is, in the context of the invention, an immunoglobulin superfamily member. Pertinent information on the human VISTA gene is found at Entrez Gene ID: 64115; HGNC ID: 30085; Genome Coordinates for assembly GRCh38:CMooo672.2: Chromosome 10: 71,747,559-71,773,498 reverse strand, and information on human VISTA protein is accessible on UniProt: Q9H7M9 (eg, Entry version of Nov. 2022) A VISTA protein in the context of the invention, typically, is approximately 50 kDa, is a type I transmembrane protein and has one IgV domain. Preferably (eg as a human VISTA protein) comprises an amino acid sequence as shown in SEQ ID NO: 1. With reference to SEQ ID NO. 1, amino acids 1 to 32 represent an N-terminal signal peptide, amino acids 33 to 194 form the extra cellular domain (SEQ ID NO. 2), amino acids 195 to 215 form the helical transmembrane (TM) region and amino acids 216 to 311 form the cytoplasmic domain. The extracellular domain (ECD) of (human) VISTA forms an Ig-like V-type domain between amino acids 33 to 168 (SEQ ID NO. 3).

The human VISTA gene is located at chromosomal position 10q22.1, and has orthologues (eg, is conserved) in many species such as in chimpanzee and other great apes, Rhesus, Cynomolgus and green monkeys, marmoset, dog, pig, cow, mouse etc. In particular, the amino acid sequence for the VISTA protein in mouse is derivable under UniProt identifier Q9D659, Entry version Nov 2023 (77.2% identical to human). The closest human paralogue to human VISTA is programmed cell death 1 ligand 1, CD274 or PD-Li (24.8% identity to human VSIR). The term "VISTA" in some embodiments of the invention may also pertain to variants of the human VISTA protein having an amino acid sequence that is substantially identical to, or of at least 70%, 75% or 80%, preferably 85%, more preferably at least 90%, 95%, 96%, 97% 98%, 99% or 100% sequence identity (such as at least 90% or 95% sequence identity) to, the amino acid sequence shown in SEQ ID NO. 1, as determined using, e.g., an algorithm described elsewhere herein, and which (preferably) retain biological activity identical or substantially identical to the respective reference VISTA. Preferred variants of VISTA protein comprise sequence variants thereof due to sequence polymorphism between and within populations of the respective species, as well as mutations compared to the wild-type sequence of VISTA (eg SEQ ID NO. 1). A preferred variant of VISTA protein is an VISTA variant (compared to eg SEQ ID NO. 1) D187E (corresponding to variant dbSNP:rs3747869). The term VISTA can mean, as applicable to the context (if not more specifically indicated), an VISTA protein (such as one described above) or an mRNA molecule encoding such an VSIR protein (VISTA RNA or VISTA mRNA).

In particular embodiments of the invention, the VISTA is human VISTA, preferably a protein comprising an amino acid sequence of: SEQ ID NO: 1, or a protein having no more than two, four, six or eight, for example no more than one, two or three, such as no more than one, amino acid substitutions, insertions or deletions compared to this sequence.

In the context of variants of VISTA, the invention includes those embodiments where a variant of VISTA is a protein comprising an amino acid sequence having at least 80%, 85%, 90%, 92% 95% or 97% sequence identity (in particular, at least 92% or 95% sequence identity) to the sequence of SEQ ID NO: 1.

In the context of other variants of VISTA, the invention also includes those embodiments where a variant of VISTA is selected from the group consisting of an ortholog (or paralog) of VISTA. In particular of such embodiments, the variant of VISTA comprises an extracellular domain (ECD) of an VISTA protein, such as an ECD of a human VISTA protein.

In particular embodiments of the present invention, an extracellular domain (ECD) of VISTA is an ECD of human VISTA protein, such as wherein the VISTA of a human VSIR protein is an amino acid sequence selected from the group consisting of: SEQ ID NO: 2 and SEQ ID NO: 3 (preferably, SEQ ID NO: 3), or an amino acid sequence having at least 80%, 85%, 90%, 92%, 95% or 97% sequence identity (preferably, at least 92% or 95% sequence identity) to these sequences, and/or having no more than two, four, six or eight, for example no more than one, two or three, such as no more than one, amino acid substitutions, insertions or deletions compared to these sequences.

In preferred embodiments therefore, the term "VISTA" shall refer to human VISTA or an above described VISTA variant. In another embodiment, either alternatively or additionally, VISTA can be understood to refer to a human VISTA.

In certain embodiments of the invention the detection of VISTA protein or mRNA is performed by analysing samples comprising an immune cell or immune cells. The term "immune cell" is art recognised to describe any cell of an organism involved in the immune system of such organism, in particular of a mammal such as a human. Leukocytes (white blood cells) are immune cells that are involved in the innate immune system, and the cells of the adaptive immune system are special types of leukocytes, known as lymphocytes. B cells and T cells are the major types of lymphocytes and are derived from hematopoietic stem cells in the bone marrow. B cells are involved in the humoral immune response, whereas T cells are involved in cell-mediated immune response. In preferred embodiments of the invention, the immune cell can be a myeloid cell eg a T cell (such as a CD4 positive T-cell), and in particular (such as when an increase in cell-mediated immune response mediated by a T cell is associated with the autoimmune disorder, such as in the context of RA) the T cell can be a cytotoxic T cell (also known as TC, cytotoxic T lymphocyte, CTL, T-killer cell, cytolytic T cell, CD4+ T-cell or killer T cell). A CTL is a T-cell that is involved in the killing of target cells, cells that are infected (particularly with viruses), or cells that are damaged in other ways, or healthy "self' cells as in the context of pathological inflammatory responses and autoimmune disorders.

As used herein, the term "biomarker" is intended to encompass a substance that is used as an indicator of a biologic state and includes genes (and nucleotide sequences of such genes), mRNAs (and nucleotide sequences of such mRNAs) and proteins (and amino acid sequences of such proteins). A preferred biomarker of the invention is VISTA, preferably human VISTA or a variant or fragment thereof. The VISTA biomarker may be detected on the basis of its gene, protein or RNA.

The method of the invention may in further preferred embodiments include, for example in step (b), detection or quantification of the expression level of one or more further biological markers are determined in the biological test sample. A "further" biomarker is in context of the invention a biomarker that is not human VISTA. On or more biological markers are selected from CTLA-4, PD-1, Lag-3, Tim-3,TIGIT, FOXP3, FR4 (folate receptor 4) and CD49b (a2 integrin chain), CD73 (ecto-5'-nucleotidase) and interleukin-10.

A treatment of an autoimmune disorder in context of the herein described invention involves preferably an administration of an immune-regulatory agent to the subject suffering from the autoimmune disorder. The term "treatment" or "treat" as used herein, refers to obtaining beneficial or desired results, including clinical results in a subject suffering from the autoimmune disorder. Beneficial or desired clinical results can include, but are not limited to, alleviation or amelioration of one or more symptoms or conditions, diminishment of extent of disease, stabilized (i.e. not worsening) state of disease, preventing spread of disease, delay or slowing of disease progression, amelioration or palliation of the disease state, diminishment of the reoccurrence of disease, and remission (whether partial or total), whether detectable or undetectable. "Treating" and "Treatment" can also mean prolonging survival as compared to expected survival if not receiving treatment. "Treating" and "treatment" as used herein also include prophylactic treatment.

More importantly, the agent administered to the subject is selected from an agent that negatively affects a T-cell mediated immune response in the subject. Such agents or therapies may be selected from immune suppressive agents and/or therapies that aim at the induction of regulatory T-cells. Treg enhancing therapies are known in the art and include the drugs rapamycin (type I diabetes), metformin (systemic lupus erythematosus) and N-Acetyl cysteine (systemic lupus erythematosus and multiple sclerosis).

Other Treg agonistic approaches in the art are based on antigen-specific Treg cell therapies and interleukin 2 (IL2). Treg cell enhancing therapies in context of the present invention are reviewed in Sharabi, A., Tsokos, M., Ding, Y. et al. Nat Rev Drug Discov 17, 823-844 (2018). https://doi.org/10.1038/nrd.2018.148, which is incorporated herein in its entirety.

A preferred Treg enhancing drug of the invention is a protein or protein complex binding to a T cell receptor suspected to be expressed in a T-cell of the subject. For example, preferably, the agent is a protein complex comprising a complex of an MHC class II protein, or an antigenic peptide binding derivative or fragment thereof, and an antigenic peptide comprising an epitope specifically mediating a binding to a T-cell receptor (or a paratope thereon).

An autoimmune disease selected in context of the invention is thus mediated by T-cells of the subject. In such cases the treatment comprises the administration of a peptide-MHC protein complex specifically binding a T-cell receptor expressed on naive T cells or T-cells regulating, driving or mediating an autoimmune disease.

A preferred treatment is a treatment of rheumatoid arthritis as described in the prior art documents WO 2021/028347. In particular included in context of the invention is a composition comprising recombinant HLA-DR/COL2 peptide complexes comprising (a) an extracellular region of an HLA-DR alpha chain comprising at least an alpha 1 domain; (b) an extracellular region of an HLA-DR beta chain comprising at least a beta 1 domain; and (c) a collagen II peptide (COL2 peptide), optionally fused to the N-terminus of the HLA-DR alpha chain or the HLA-DR beta chain by a linker peptide, preferably to the HLA-DR beta chain; wherein the COL2 peptide comprises the amino acid sequence selected from the group consisting of AGFKGEQGPKG (SEQ ID NO: 4), AGFKGEQGPXG (SEQ ID NO: 5), AGFKGEXGPKG (SEQ ID NO: 6), AGFKGXQGPKG (SEQ ID NO: 7), AGFKXEQGPKG (SEQ ID NO: 8), AGFKGEXGPXG (SEQ ID NO: 9), AGFKGXQGPXG (SEQ ID NO: 10) and AGFKXEQGPXG (SEQ ID NO: 11), and wherein the HLA-DR/COL2 peptide complexes comprise a chondroitin-binding peptide at the C-terminal end of the polypeptide comprising the HLA-DR alpha chain and/or the HLA-DR beta chain. In that context the autoimmune disorder treated with such complexes is preferably selected from chronic inflammatory diseases in human patients, particularly chronic inflammatory joint disease and/or arthritis. Preferably the composition for use in treating chronic inflammatory disease is selected from the group consisting of rheumatoid arthritis, osteoarthritis, psoriatic arthritis, non-radiographic axial spondyloarthritis, ankylosing spondylitis, juvenile idiopathic arthritis, relapsing polychondritis, systemic lupus erythematosus, Lyme disease, Meniere diseases, Autoimmune inner ear disease (AIED), or Still's disease.

The term "biological test sample" is selected from whole blood, plasma, serum, synovial fluid, saliva, urine, stool, tears, hair, skin, any other bodily fluid, tissue samples (e.g., biopsy), and cellular extracts thereof. Thus, a "biological test sample" may be selected from any tissue sample or a body fluid sample. A tissue sample includes (but is not limited to) buccal cells, a brain sample, a skin sample or organ sample (e.g. liver). The term "body fluid" refers to all fluids that are present in the body including but not limited to blood, plasma, serum, synovial fluid, lymph, urine, saliva or cerebrospinal fluid. The biological sample may also be obtained by subjecting it to a pre-treatment if necessary, for example, by homogenizing or extracting. Such a pre-treatment may be selected appropriately by those skilled in the art depending on the biological sample to be subjected. A preferred biological test sample is a synovial fluid sample.

The diagnostic/monitoring methods of the invention in all aspects or embodiments may preferably be an *ex-vivo* or *in-vitro* method.

In some embodiments, the biological test sample provided in step (a) is a sample of a subject that before the biological test sample is taken, has received or still receives a treatment of the autoimmune disorder.

Detection of the presence or absence or determination of any amount or level of the biomarker of the invention can be realized using any known prior art method. Such detection and quantification methods are dependent on the nature of the biomarker and therefore differ dependent on whether the biomarker (preferably VISTA) is detected based on VISTA protein, VISTA RNA or any specific genetic alteration in the VISTA gene region. For example, the level or expression of VISTA protein is determined immunological, for example using a VISTA specific antibody, which may either be labelled fluorescent or is detectable by a fluorescently labelled secondary antibody; or wherein the expression level of the VISTA RNA is detected using Polymerase Chain Reaction (PCR) based methods such as Reverse Transcribed (RT) PCR, more preferably quantitative RT-PCR (qRT-PCR), or RNA sequencing.

Generally, immunological detection of VISTA protein may be performed in preferred embodiments using immunostaining of cell- or tissue samples, Western blotting, ELISA or a fluorescence-activated cell scanning (FACS). Such immunological methods involved the use of VISTA specific antibodies.

As used herein, the term "antibody" may be understood in the broadest sense as any immunoglobulin (Ig) that enables binding to its epitope. Full length "antibodies" or "immunoglobulins" are generally heterotetrameric glycoproteins of about 150 kDa, composed of two identical light and two identical heavy chains. Each light chain is linked to a heavy chain by one covalent disulphide bond, while the number of disulphide linkages varies between the heavy chain of different immunoglobulin isotypes. Each heavy and light chain also has regularly spaced intrachain disulphide bridges. Each heavy chain has an amino terminal variable domain (VH) followed by three carboxy terminal constant domains (CH). Each light chain has a variable N-terminal domain (VL) and a single C-terminal constant domain (CL). The VH and VL regions can be further subdivided into regions of hypervariability, termed complementarity determining regions (CDR), interspersed with regions that are more conserved, termed framework regions (FR). Each VH and VL is composed of three CDRs and four FRs, arranged from amino-terminus to carboxy-terminus in the following order: FR1, CDR1, FR2, CDR2, FR3, CDR3, FR4. The variable regions of the heavy and light chains contain a binding domain that interacts with an antigen. The constant regions of the antibodies may mediate the binding of the immunoglobulin to cells or factors, including various cells of the immune system (e.g., effector cells) and the first component (C1q) of the classical complement system. Other forms of antibodies include heavy-chain antibodies, being those which consist only of two heavy chains and lack the two light chains usually found in antibodies. Heavy-chain antibodies include the hcIgG (IgG-like) antibodies of camelids such as dromedaries, camels, llamas and alpacas, and the IgNAR antibodies of cartilaginous fishes (for example sharks). And yet other forms of antibodies include single-domain antibodies (sdAb, called Nanobody by Ablynx, the developer) being an antibody fragment consisting of a single monomeric variable antibody domain. Single-domain antibodies are typically produced from heavy-chain antibodies, but may also be derived from conventional antibodies.

An antibody in context of the present invention is preferably an antibody, or an antigen binding fragment thereof, that specifically binds to a VISTA protein, or a protein of a VISTA variant as described elsewhere herein. Preferably the antibody of the invention is capable of specifically binding to, and therefore facilitating the detection of, a protein comprising an amino acid sequence shown in SEQ ID Nos: 1 to 3.

In some embodiments, determination and/or diagnosis method of the invention can comprise, such as in a further step, comparing the detected amount (or activity of) of (eg protein or mRNA of) the applicable biomarker (ie VISTA or a variant thereof) with a standard or cut-off value; wherein a detected amount greater than the standard or cut-off value indicates a phenotype (or a risk of developing a phenotype) that is associated with the undesired presence of VISTA-positive T cells (or cells positive for a variant of VISTA) and/or that is associated with cellular resistance against the cell-mediated immune response in the subject and/or is associated with (eg aberrant) expression or activity of VISTA (or the variant) in the subject. Such a standard or cut-off value may be determined from the use of a control assay, or may be pre-determined from one or more values obtained from a study or a plurality of samples having known phenotypes, such as such assays performed in the experimental section herein below. For example, a cut-off value for a diagnostic test may be determined by the analysis of samples taken from patients in the context of a controlled clinical study, and determination of a cut-off depending on the desired (or obtained) sensitivity and/or specificity of the test.

Examples of methods useful in the detection of (such as the presence or absence of, or an amount of) the applicable biomarker (i.e. the VISTA or variant thereof) include immunoassays, such as the enzyme linked immunosorbent assay (ELISA), flow cytometry based assay, such as fluorescent activated cell sorting (FACS), and the radioimmunoassay (RIA), which employ antibodies (e.g. VISTA specific antibodies) such as an antibody or an antigenbinding fragment thereof, that specifically binds to such applicable biomarker (preferably VISTA protein or a variant thereof).

For such methods, a monoclonal antibody or a polyclonal antibody may be employed. Examples of monoclonal antibodies of VISTA are well known in the art. The term "polyclonal antibody" as used herein refers to a mixture of antibodies which are genetically different since produced by plasma cells derived from multiple somatic recombination and clonal selection events and which, typically, recognise a different epitope of the same antigen.

Alternatively, the presence of the applicable biomarker (ie VISTA or variant thereof) may be detected by detection of the presence of mRNA that encodes such applicable biomarker, or fragments of such mRNA. Methods to detect the presence of such mRNA (or fragments) can include, PCR (such as quantitative RT-PCR), hybridisation (such as to Illumina chips), nucleicacid sequencing etc. Such methods may involve or comprise steps using one or more nucleic acids as described herein, such as PCR primers or PCR probes, or hybridisation probes, that bind (e.g. specifically) to such mRNA.

For such detection, determination or diagnostic applications, the antibody or nucleic acid, typically, will be labelled with a detectable labelling group. In general, labelling groups fall into a variety of classes, depending on the assay in which they are to be detected: a) isotopic labels, which may be radioactive or heavy isotopes; b) magnetic labels (e.g., magnetic particles); c) redox active moieties; d) optical dyes; enzymatic groups (e.g. horseradish peroxidase, betagalactosidase, luciferase, alkaline phosphatase); e) biotinylated groups; and f) predetermined polypeptide epitopes recognised by a secondary reporter (e.g., leucine zipper pair sequences, binding sites for secondary antibodies, metal binding domains, epitope tags, etc.). Suitable labelling groups include, but are not limited to, the following: radioisotopes or radionuclides (e.g., 3H, 14C, 15N, 35S, 90Y, 99Tc, 111In, 125I, 131I), fluorescent groups (e.g., FITC, rhodamine, lanthanide phosphors), enzymatic groups (e.g., horseradish peroxidase, betagalactosidase, luciferase, alkaline phosphatase), chemiluminescent groups, biotinylated groups, or predetermined polypeptide epitopes recognised by a secondary reporter (eg, leucine zipper pair sequences, binding sites for secondary antibodies, metal binding domains, epitope tags). In some embodiments, the labelling group is coupled to the antibody or nucleic acid via spacer arms of various lengths to reduce potential steric hindrance. Various methods for labelling proteins are known in the art and may be used. For example, the antibody or nucleic acid may be labelled with a secondary reporter (e.g., leucine zipper pair sequences, binding sites for secondary antibodies, metal binding domains, epitope tags, etc.).

Accordingly, in particular embodiments of the detection/diagnostic methods (or the kits therefor), the means (e.g. antibody or nucleic acid) for the detection (e.g. detector) of protein or mRNA of the applicable biomarker (e.g. VISTA), is labelled, for example is coupled to a detectable label. The term "label" or "labelling group" refers to any detectable label, including those described herein.

In certain embodiments, the detection/diagnostic methods of the invention involve an immunohistochemistry (IHC) assay or an immunocytochemistry (IC) assay. The terms "IHC" and "ICC" are art recognised and include the meanings of techniques employed to localise antigen expression that are dependent on specific epitope-antibody interactions. IHC typically refers to the use of tissue sections, whereas ICC typically describes the use of cultured cells or cell suspensions and is as such preferred. In both methods, positive staining is typically visualised using a molecular label (eg, one which may be fluorescent or chromogenic). Briefly, samples are typically fixed to preserve cellular integrity, and then subjected to incubation with blocking reagents to prevent non-specific binding of the antibodies. Samples are subsequently typically incubated with primary (and sometimes secondary) antibodies, and the signal is visualised for microscopic analysis.

Accordingly, such embodiments of the detection/diagnostic methods of the invention may include a step of preparing a subject IHC or ICC preparation tissue or cells (such as those present in the biological samples obtained from a subject); and preferably wherein the detection of binding of an antibody to the applicable biomarker (i.e. VISTA or a variant thereof) expressed by the tissues of cells said IHC or ICC preparation indicates: (i) a treatment susceptibility to an autoimmune disease, disorder or condition (or a chance of treatment success) involving enhancement of T regulatory cell activity.

The antibody (specifically binding to VISTA, or a variant thereof) used in such methods is, preferably, validated. For example, the antibody is validated to (detectably) bind to the applicable biomarker (ie VISTA or a variant thereof) expressed by the cells and/or tissues of said validation IHC or ICC preparation, but does not (detectably) bind to a control IHC or ICC preparation of control cells and/or tissues that do not express such applicable biomarker. Preferably, said control cells are gene knock-down or gene knock-out cells and/or tissues for the applicable biomarker (ie VISTA or a variant thereof); more preferably, wherein said gene knock-down or gene knock-out cells and/or tissues are siRNA or shRNA gene knock-down or gene knock-out for such applicable biomarker.

In **a third aspect,** the invention pertains to a compound or composition for use in the treatment of an autoimmune disorder in a subject, wherein the compound and composition is immune-regulatory and negatively affects a T-cell mediated immune response in the subject when administered, and wherein the treatment further comprises obtaining a biological test sample of the subject at least once before, during and/or subsequent to the administration of the compound or composition, and wherein the subject is stratified as being successfully treated or being susceptible to the treatment by a method according to the herein elsewhere described first and second aspects, using the biological test sample of the subject.

In an embodiment of the third aspect, it is preferred that if an increased level in (i) and/or an increased number/percentage in (ii) compared to the respective reference indicates that the subject is further administered with the compound or composition.

In another embodiment of the third aspect, it is preferred that if a decreased level in (i) and/or a decreased number/percentage in (ii) compared to the respective reference indicates that the subject is further administered with the compound or composition

Treatments are as described herein above, including the suggested agents and therapies enhancing T regulatory cell functions and activity.

The terms "of the [present] invention", "in accordance with the invention", "according to the invention" and the like, as used herein are intended to refer to all aspects and embodiments of the invention described and/or claimed herein.

As used herein, the term "comprising" is to be construed as encompassing both "including" and "consisting of', both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

It is to be understood that application of the teachings of the present invention to a specific problem or environment, and the inclusion of variations of the present invention or additional features thereto (such as further aspects and embodiments), will be within the capabilities of one having ordinary skill in the art in light of the teachings contained herein.

Unless context dictates otherwise, the descriptions and definitions of the features set out above are not limited to any particular aspect or embodiment of the invention and apply equally to all aspects and embodiments which are described.

All references, patents, and publications cited herein are hereby incorporated by reference in their entirety.

### BRIEF DESCRIPTION OF THE FIGURES AND SEQUENCES

The figures show:

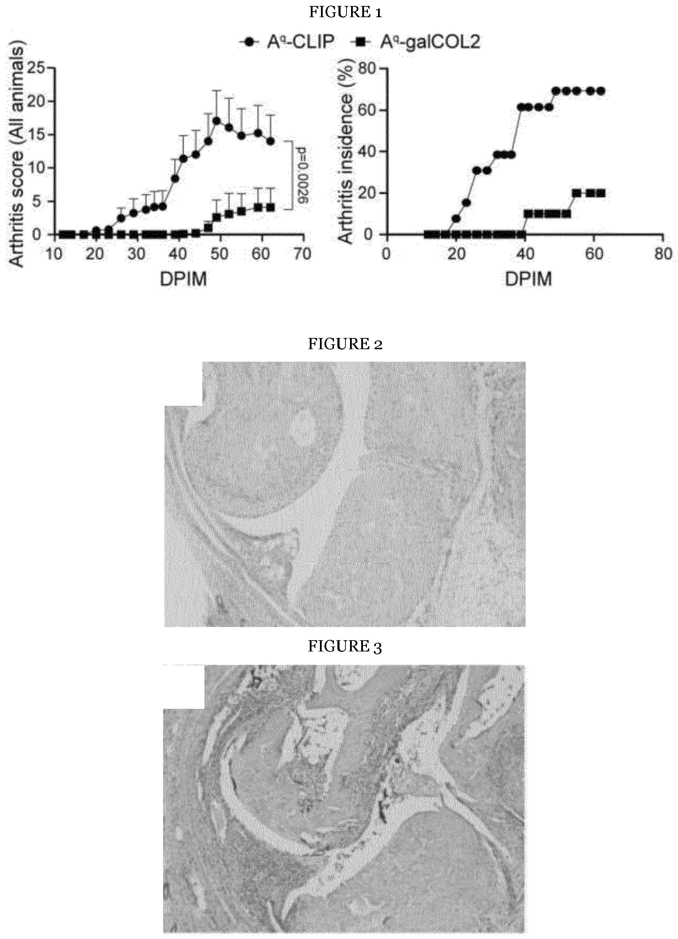

**Figure 1****:** Tolerogenic collagen peptide-based vaccine attenuates autoimmune pathologies (CIA, DTH, CAIA) in a T-cell-dependent manner. CIA in vaccinated mice as assessed by mean clinical score of arthritis severity (left panel) and incidence of arthritis (percentage of affected mice [right panel]) on different days post-immunization (DPIM). QB mice were immunized with 100 µg of COL2 on day 0 and boosted on day 35 with 50 µg of COL2. On day 7, mice were vaccinated s.c. with Aq-peptide complexes using osmotic pumps (n=10 for A^{q}-galCOL2, n=13 for A^{q}-CLIP).
**Figure 2****:** Histological examination of an ankle joint from A^{q}-galCOL2-treated QB mice.
**Figure 3****:** Histological examination of an ankle joint from A^{q}-CLIP treated mice demonstrating cell infiltration and cartilage/bone destruction.

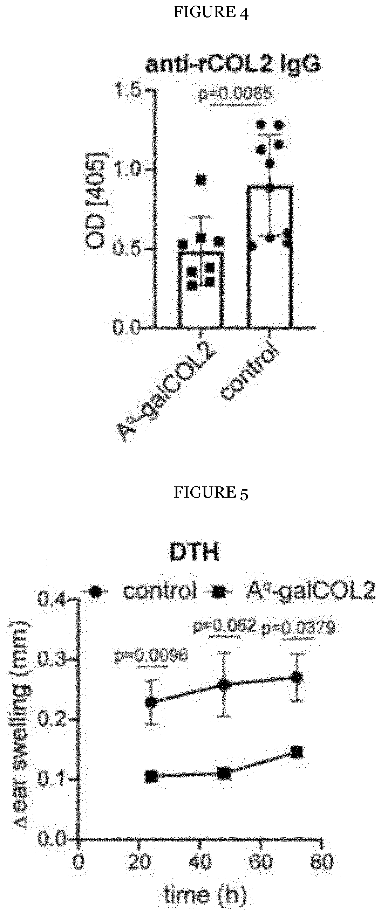

**Figure 4****:** Anti-COL2 IgG serum levels assessed at day 35 during CIA by ELISA.
**Figure 5****:** DTH reactions (ear swelling) following treatment with A^{q}-galCOL2 complexes.

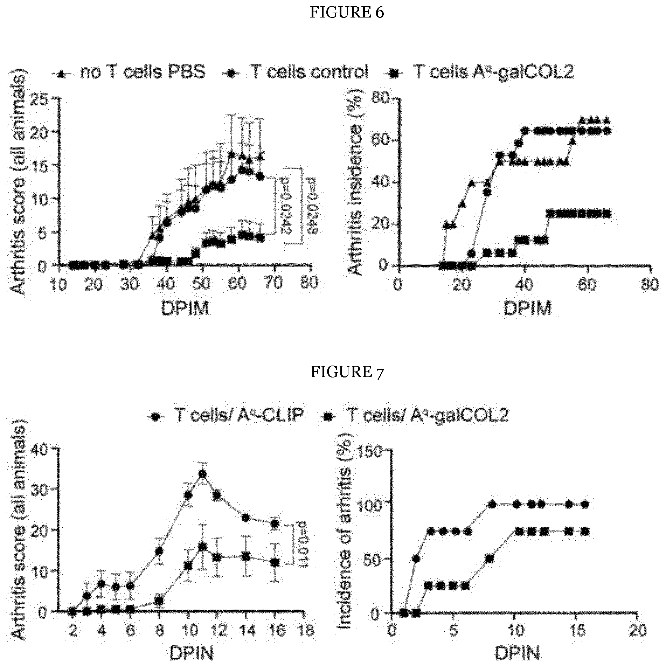

**Figure 6****:** Mean clinical score of arthritis severity (left panel) and incidence (right panel) following transfer of T cells from Aq-galCOL2-treated and A^{q}-CLIP-treated QB mice (n=5 per group).
**Figure 7****:** CAIA following transfer of CD4+T cells from A^{q}-galCOL2-treated and Aq-CLIP-treated BC mice (n=5 per group). Mice were immunized with COL2 on day 5 and vaccinated either with 100 µg A^{q}-galCOL2 or Aq-CLIP using osmotic pumps. On day 15 purified CD4+ T cells were transferred to *BQ.Cia9i* recipients, which were injected with the arthritogenic CAIA cocktail (M2139 and ACC1 anti-COL2 antibodies) on the same day as the T-cell transfer. Results are expressed as mean ± SEM. DPIN, days post-injection.

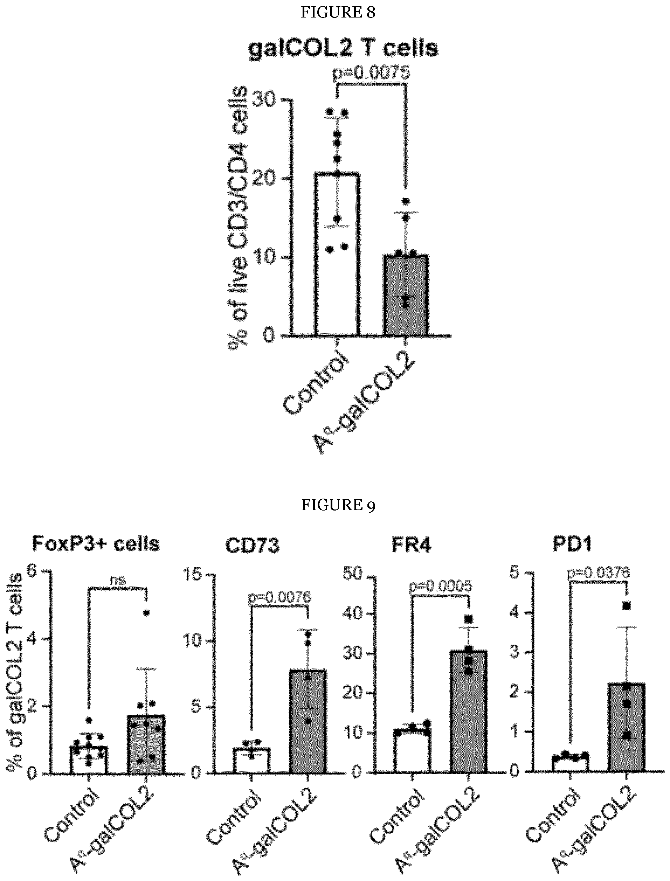

**Figure 8****:** Treatment with A^{q}-galCOL2 complexes reduces frequencies of galCOL2 specific T cells. HCQ3tg mice were immunized with COL2/CFA, at day 5 post-immunization osmotic pumps filled with A^{q}-peptide complexes were implanted (2001D, 100 µg). Day 12 postimmunization T cells in dLN were analyzed with galCOL2 peptide/A^{q} tetramer staining using flow cytometry.
**Figure 9****:** Vaccination with the Aq-galCOL2 complexes induces tolerogenic phenotype in galCOL2-specific CD4+ T cells. Upregulation of Foxp3, CD73, FR4, and PD1 was assessed by either galCOL2 peptide/A^{q} tetramer staining and immunophenotyping or a congenic marker CD45.1.

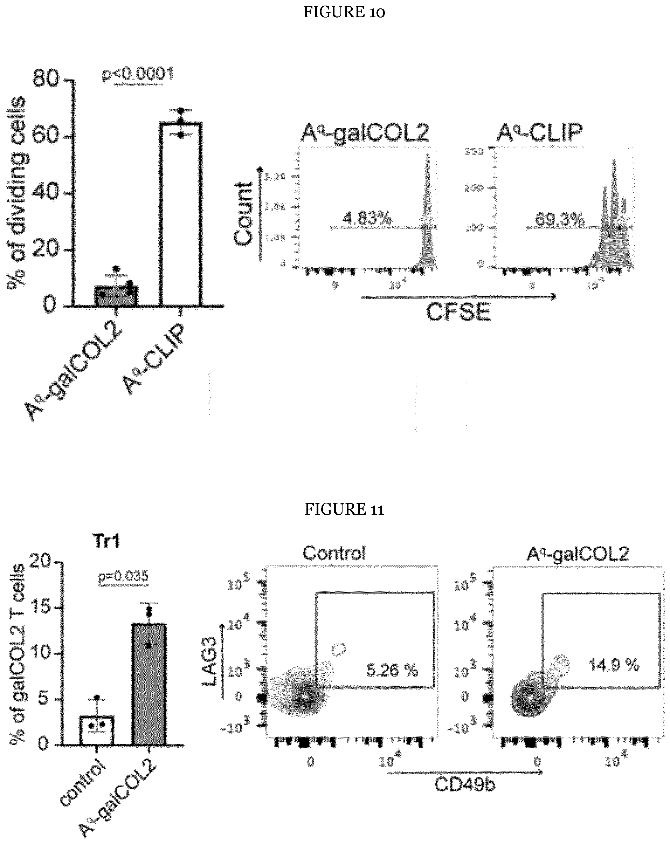

**Figure 10****:** *BQ.HCQ3tg.Rag1^{-l-}* T cells from the vaccinated mice suppress anti-CD3/CD28-induced polyclonal T cell proliferation. GalCOL2-specific CD4+ T cells from the vaccinated BQ.HCQ3tg.Rag1-/- mice (suppressor cells) were co-cultured with 1× 105 purified CD45.2 expressing CFSE-labeled CD4+ T cells from BQ mice (responder cells) in vitro at a ratio of 1:3. FACS analysis was performed three days after stimulation with anti-CD3/CD28 Abs. Quantification (left panel) and representative gating (right panel) are shown.
**Figure 11****:** Induction of a unique T regulatory phenotype by A^{q}-galCOL2 complexes in *BQ.HCQ3tg.MMCtg* mice. *BQ.HCQ3tg.MMCtg* mice were immunized with COL2/CFA, at day 5 post-immunization osmotic pumps filled with Aq-peptide complexes were implanted (2001D, 100 µg). Day 12 post-immunization, the expression of CD49b and LAG3 (markers for Tr1 phenotype) within the splenic gal-COL2/A^{q} tetramer+ CD4+ T cells was analyzed by flow cytometry (gated on live CD3+CD4+ galCOL2/A^{q} tetramer+ cells).

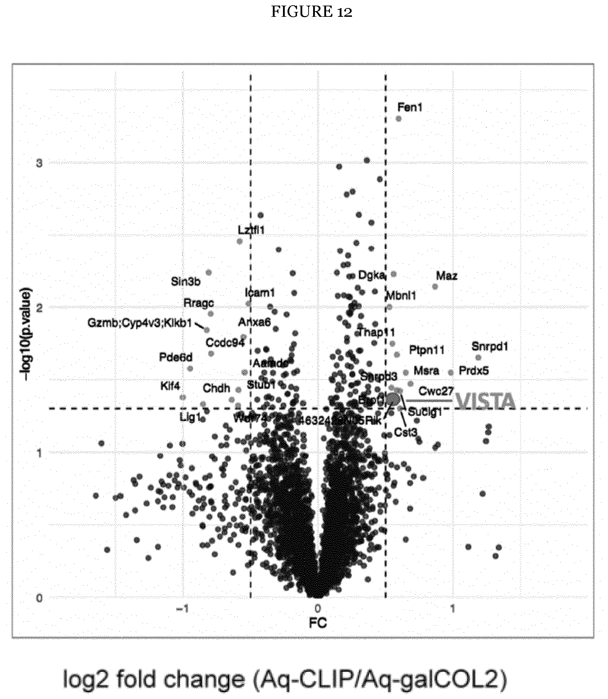

**Figure 12****:** shows e Volcano plot comparing the proteomic profile of the galCOL2-specific T cells treated with A^{q}-CLIP with the galCOL2-specific T cells treated with Aq-galCOL2 complex. *BQ.HCQ.3 tg* mice were immunized with COL2/CFA. Day five post-immunization osmotic pumps (model 2001D) filled either with 100 µg of Aq-galCOL2 complex (n=3) or control A^{q}-CLIP complex (n=4) were implanted. On day 10 post-immunization iLN cells were collected, Ag-specific CD4+ T cells were labelled with galCOL2/A^{q} tetramers and sorted by BD FACS Aria flow FACS sorter. The sorted cells were subjected to LC-MS/MS. Proteins involved in T cell tolerance and T cell effector functions that were significantly and substantially up- or down-regulated are highlighted in blue. Rrgac, Ras-related GTP-binding protein C, Dgka, Diacylglycerol kinase alpha; Vista, V-domain immunoglobulin suppressor of T cell activation.

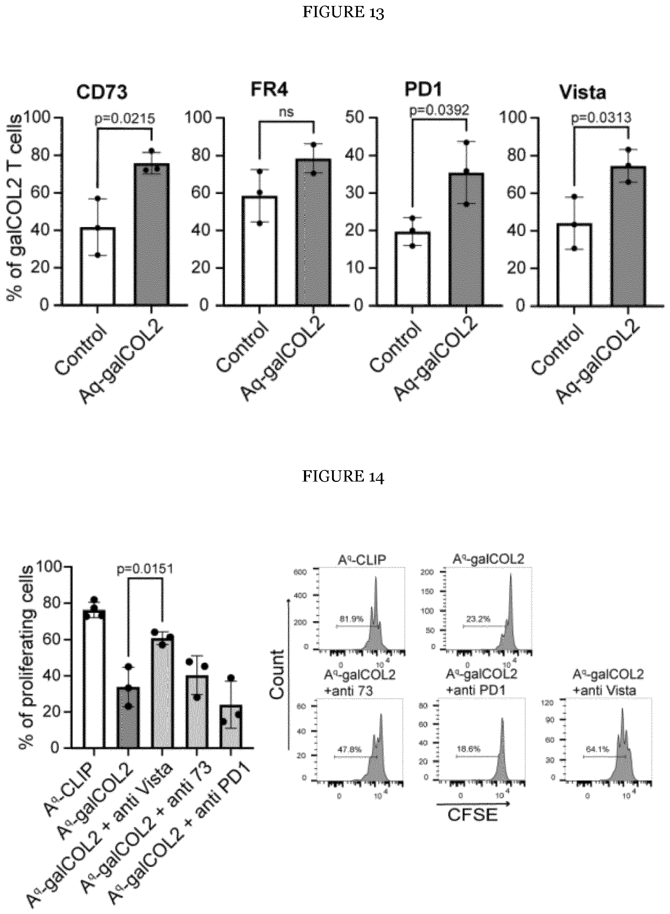

**Figure 13****:** Vaccination with A^{q}-galCOL2 leads to elevated expression of VISTA, CD73, FR4, and PD1 on gal-COL2 specific T cells from wt mice. BQ wt mice were immunized with COL2/CFA, at day 5 post-immunization osmotic pumps filled with Aq-peptide complexes were implanted (2001D, 100 µg, n=3 per group). Day 10 postimmunization splenocytes and dLN cells were recovered, stained with the PE-labelled galCOL2/Aq tetramer and enriched using anti-PE magnetic beads. The phenotypic analysis of the enriched galCOL2-specific CD4+ T cells were performed by flow cytometry (gated on live CD4+ galCOL2/A^{q} tetramer+ cells).
**Figure 14****:** VISTA is required for the suppressive effect of tolerogenic galCOL2-specific cells. Osmotic pumps filled with A^{q}-peptide complexes were implanted s.c. (2001D, 100 µg, n=3 per group) to BQ.HCQ3tg mice. Day 6 after implantation splenocytes were recovered, CD4+T cells were purified and co-cultured with purified CFSE-labelled CD4+ T cells from BQ mice in the presence or in the absence of the blocking antibodies targeting VISTA, CD73 or PD1. FACS analysis was performed three days after stimulation with anti-CD3/CD28 Abs. Quantification (left panel) and representative gating (right panel) are shown. Results are expressed as mean ± SD.

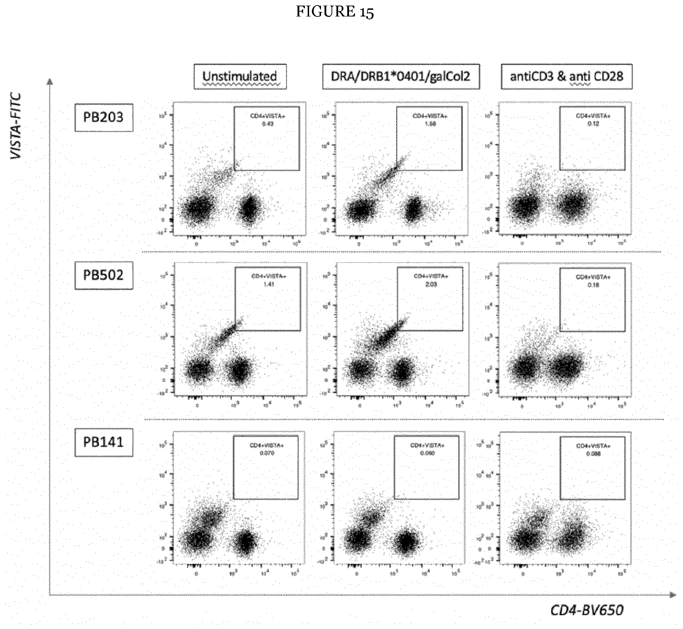

**Figure 15****:** Peripheral mononuclear blood cells (PBMCs) from HLA DRB1^{∗}0401 RA patients were seeded in a 24-well plate (1×10⁶ cells, 1 ml/well) and stimulated in TexMACS medium (Milteny Biotec) with 1% penicillin/streptomycin (Gibco) for 24 hours under the conditions following conditions. For the stimulation with the soluble DR4-galCol2 construct, the wells were previously pre-incubated with 15% FCS/medium for 6 hours and then washed with PBS (Gibco). As controls, the PBMCs were stimulated with coated with anti-human CD3 (αCD3, Ultra-LEAF purified anti-human CD3, Clone OKT3, BioLegend) and soluble anti-human CD28 (αCD28, 1 µg/ml, LEAF purified anti-human CD28, clone: CD28.2, BioLegend). The precoating of the wells with the anti-CD3 antibody was performed by incubation at a concentration 1 µg/ml αCD3 (1 ml/well) for 6 hours followed by washing (2x) with PBS. In the control condition, the cells remained untreated. To test the active substance, cells were stimulated with the recombinant DRA/DRB1COL2₂₅₉₋₂₇₄ peptide complex at a concentration of 5 µg/ml in medium.
   After 24 hours the cells were detached from the plate and transferred to FACS tubes and rinsed with 1 ml PBS. The samples were then centrifuged at 1900 rpm for 5 minutes. The cell pellet was incubated with 2 µl of human FcR blocker (Milteny Biotec) at 4° C. for 10 minutes. Subsequently, the cells were washed with 2 ml PBS. The resuspended cell pellet was stained with 2µl anti-human VISTA, FITC (Invitrogen, Clone: B7H5DS8), 1µl antihuman CD4,BV650 (BioLegend, Clone:OKT4) and 0.2µl zombie NIR (BioLegend) at 4°C in the dark for 20 min followed by washing of cells in 2 ml FACS wash (PBS+0.5% BSA). Finally, the CD4+ T cells were then analyzed for VISTA staining by flow cytometry (FACS Fortessa [BD; Franklin Lakes, NJ], in the single cell suspension using FlowJo Software [Treestar; Ashland, OR]) by gating on the CD4+ cells in the live population according to live/dead marker (Zombie NIR, Biolegend #77184) and corresponding to Fluorescence Minus One (FMO) controls.
   The upper two rows show for two samples (P203 and P502) a specific effect of DR4-galCol2 on an expansion of the VISTA positive subset of CD4+T cells depicted in the highlighted field in the upper right quadrant of the panels (containing the double positive cells) compared to unstimulated or anti-CD3/anti-CD28 antibody activated PBMCs. The bottom row depicts the result of the stimulation of PMNCs from sample P141 that did not exhibit VISTA upregulation on T cells in response to in vitro challenge with DR4-galCOL2 vaccine. Accordingly, the data shows the specific upregulation of VISTA on a subset of CD4+ cells in DR4-galCOL2 stimulated cells, however, at an extent that might differ in between individual patients.
   **SEQ ID NO: 1** - shows human V-type immunoglobulin domain-containing suppressor of T-cell activation amino acid sequence 
MGVPTALEAGSWRWGSLLFALFLAASLGPVAAFKVATPYSLYVCPEGQNVTLTCRLLGPVDKG
HDVTFYKTWYRSSRGEVQTCSERRPIRNLTFQDLHLHHGGHQAANTSHDLAQRHGLESASDH
HGNFSITMRNLTLLDSGLYCCLVVEIRHHHSEHRVHGAMELQVQTGKDAPSNCVVYPSSSQDS
ENITAAALATGACIVGILCLPLILLLVYKQRQAASNRRAQELVRMDSNIQGIENPGFEASPPAQGI
PEAKVRHPLSYVAQRQPSESGRHLLSEPSTPLSPPGPGDVFFPSLDPVPDSPNFEVI
   **SEQ ID NO: 2** - shows an ECD of human VISTA protein; UniProt identifier Q9H7M9): 
FKVATPYSLYVCPEGQNVTLTCRLLGPVDKGHDVTFYKTWYRSSRGEVQTCSERRPIRNLTFQD
LHLHHGGHQAANTSHDLAQRHGLESASDHHGNFSITMRNLTLLDSGLYCCLVVEIRHHHSEH
RVHGAMELQVQTGKDAPSNCVVYPSSSQDSENITAA
   **SEQ ID NO: 3** - shows the Ig-like V-type domain of human VISTA protein; UniProt identifier Q9H7M9):

FKVATPYSLYVCPEGQNVTLTCRLLGPVDKGHDVTFYKTWYRSSRGEVQTCSERRPIRNLTFQD
LHLHHGGHQAANTSHDLAQRHGLESASDHHGNFSITMRNLTLLDSGLYCCLVVEIRHHHSEH
RVHGAMELQV

### EXAMPLES

Certain aspects and embodiments of the invention will now be illustrated by way of example and with reference to the description, figures and tables set out herein. Such examples of the methods, uses and other aspects of the present invention are representative only, and should not be taken to limit the scope of the present invention to only such representative examples.

The examples show:

### Example 1: A^{q}-galCOL2 complexes ameliorate autoimmune arthritis.

It was previously shown that two intravenous (i.v.) injections with A^{q} molecules loaded with the galCOL2₂₅₉₋₂₇₃ peptide (A^{q}-galCOL2) mediates protection from arthritis in mice. Based on *in vitro* and in *vivo* stability experiments (not shown), the inventors used osmotic ALZET pumps instead of i.v. injections to prolong the availability of A^{q}-galCOL2. Consistent with the previous data, subcutaneous (s.c.) administration of A^{q}-galCOL2 complexes using osmotic pumps significantly reduced the severity and incidence of CIA as well as considerably delayed the onset of the arthritis (Fig. 1). In this and in further in *vivo* experiments (if not stated differently), A^{q}-CLIP complex released by osmotic pumps served as a negative control.

The arthritis scoring data were confirmed by histological analysis of the paws at the end of the experiment (Fig. 2 and 3). Furthermore, anti-COL2 IgG antibody responses were reduced in the A^{q}-galCol2-treated group as compared with the control group treated with A^{q}- CLIP (Fig. 4).

### Example 2: A^{q}-galCOL2 complexes target antigen-specific T cells.

Since Thi-mediated immunity is involved in RA, and in animal models for RA, the inventors investigated the potential of A^{q}-galCOL2 to downregulate COL2-specific Thi cell-mediated immune responses. The inventors employed a model of delayed-type hypersensitivity (DTH) reaction induced by COL2 sensitization and challenge, in which antigen-specific Thi cells are required for eliciting the hypersensitivity reaction. COL2 challenge markedly increased the thickness of ears in COL2-sensitized mice and the ear swelling was attenuated by A^{q}-galCOL2 administration (Fig. 5), indicating a functional inactivation of COL2-specific Th 1 cells.

Next, adoptive T-cell transfer experiments were performed to test whether T cells from the mice treated with A^{q}-galCOL2 could transfer the suppressive effect. Three groups of *QB* mice (donors, 5 mice per group) received Aq-galCOL2 in phosphate-buffered saline (PBS), A^{q}-CLIP in PBS, or PBS only at day 0 via osmotic pumps. At the same time (day 0), another group of QB mice (recipients) were immunized with COL2 in complete Freund's adjuvant (CFA). Seven days after the Aq-galCOL2 injection, purified CD4+ T cells from the spleens and draining lymph node (dLN) of the donors were transferred i.v. to the immunized recipients. T cells from the mice that received Aq-galCOL2 complexes were effective at preventing the induction and development of arthritis (Fig. 6).

Finally, it was explored if T cells from the A^{q}-galCOL2-vaccinated mice could suppress arthritis independent of other T or B cells. Thus, collagen antibody-induced arthritis (CAIA), was induced by intraperitoneal injection of the arthritogenic cocktail consisting of M2139 and ACC1 monoclonal antibodies, directed to COL2 and citrullinated COL2, and the disease was boosted with lipopolysaccharide on day 6 post-injection. *BQ* donors were immunized with COL2 and treated with either A^{q}-galCOL2 or Aq-CLIP on day 5 post-immunization. On day 15, postimmunization CD4+T cells were purified and transferred i.v. to naive recipients, and the arthritogenic antibody cocktail was injected the same day following the T-cell transfer. As shown in Fig. 7, T cells from the A^{q}-galCOL2-treated donors attenuated CAIA severity and delayed onset of the disease. Thus, A^{q}-galCOL2 treatment generates regulatory cells from the natural T-cell pool that can actively suppress the joint inflammation mediated by pathogenic autoantibodies.

### Example 3: Treatment with Aq-galCOL2 expands antigen-specific CD4+ regulatory T cells.

Since Thi-mediated immunity is involved in RA and in animal models for RA the inventors investigated the potential of A^{q}-galCOL2 to downregulate COL2-specific Thi cell-mediated immune responses. The inventors employed a model of delayed-type hypersensitivity (DTH) reaction induced by COL2 sensitization and challenge, in which antigen-specific Thi cells are required for eliciting the hypersensitivity reaction. COL2 challenge markedly increased the thickness of ears in COL2-sensitized mice and the ear swelling was attenuated by A^{q}-galCOL2 administration, indicating a functional inactivation of COL2-specific Thi cells.

The inventors then characterized tolerogenic T cells that mediate the vaccination effect. To evaluate the phenotype of COL2-specific T cells, the inventors used *BQ* mouse lines expressing a transgenic (tg) αβTCR (*HCQ3tg*) that specifically recognizes the glycosylated form of the COL2₂₅₉₋₂₇₃ peptide in the context of Aq and peptide/MHCII tetramers. First, the inventors found that administration of A^{q}galCOL2 to BQ.HCQ3tg mice lead to reduction of the number of galCOL2-specific T cells (Fig. 8). The galCOL2-specific T cells from the BQ.HCQ3tg mice treated with Aq-galCOL2 had increased expression of cell surface markers associated with a regulatory phenotype (CD73, folate receptor 4 [FR4], programmed death-i [PD-1], and forkhead box P3 [FoxP3]) (Fig. 9). In fact, the phenotypes of these cells were similar to those observed in *BQ.HCQ3tg.MMCtg* mice. *MMCtg* mice express the immunodominant T-cell epitope of rat/human COL2 (i.e., with 266E). Consequently, *MMCtg* mice display T-cell tolerance when immunized with rat COL2 and have a reduced susceptibility to arthritis compared with nontolerized wild type (wt) mice.

The inventors performed functional assays to examine whether COL2-specific T cells treated with A^{q}-galCOL2 exerted a dominant suppressive effect on activated polyclonal T cells. HCQ3tg recombination activating gene 1 (RAG1)-deficient mice, which only have HCQ3-specific T cells, were treated with A^{q}-galCOL2. Four days post-vaccination, galCOL2-specific T cells from the spleens of *BQ.HCQ3tg.RAG1^{-l-}* mice were enriched and co-cultured with purified carboxyfluorescein N-succinimidyl ester (CFSE)-labelled CD4+ T cells from wild type *BQ* mice activated with anti-CD3 and anti-CD28 antibodies. After 3 days of cell culture, proliferation of the polyclonal T cells was assessed by fluorescence-activated cell sorting (FACS). As shown in Fig. 10, tolerogenic galCOL2-specific T cells generated in *vivo* by administration of A^{q}-galCOL2 efficiently suppressed activation and proliferation of noncognate T cells.

Of note, when A^{q}-galCOL2 complexes were administered to *BQ.HCQ3tg.MMCtg* mice, a Tr1-regulatory phenotype (LAG3 and CD49b double positive) was induced on the galCOL2 T cells (Fig. 11). To provide a more systematic characterization of the regulatory phenotype of galCOL2-specific CD4+T cells elicited by treatment with A^{q}-galCOL2, the inventors compared proteomic changes in these cells (Fig. 12). GalCOL2-specific CD4+ T cells from *BQ.HCQ.3tg* mice, immunized with COL2/CFA and treated with the Aq-galCOL2 *ex vivo,* were FACS sorted with galCOL2/A^{q} tetramers and analyzed by liquid chromatography with tandem mass spectrometry (LCMS/MS). The results confirmed the anergic phenotype in galCOL2-specific CD4+ T cells from A^{q}-galCOL2-vaccinated mice (reflected by CD73, FR4 overexpression, and hyporesponsiveness to TCR stimulation), and revealed an overexpression of diacylglycerol kinase α(DGKα), an enzyme critical for anergy induction via depletion of diacylglycerol, and a decreased expression of Ras-related GTP-binding protein C (Rragc), a small G-protein that mediates a crucial step in the activation of the mechanistic target of rapamycin (mTOR) signaling cascade, which is reported to drive T-cell activation and differentiation into Thi, Th2, and Th17 effector T-cell lineages. Conversely, inactivation of mTOR in T cells affects their regulatory functions, which is also seen in galCOL2-specific T cells in response to A^{q}galCOL2 treatment. Also, an enhanced expression of a surface molecule, V-type immunoglobulin domain-containing suppressor of T-cell activation (VISTA) was found, that may crucially contribute to tolerogenic potency of Aq-galCOL2-treated antigen-specific CD4+T cells.

VISTA is a potent negative regulator of T-cell responses that actively imposes quiescence on naive T and myeloid cells and inhibits T-cell activation and cytokine production. Moreover, VISTA is required for inducible Treg generation and stability, as genetic deficiency of VISTA on the T cells reduces inducible Treg differentiation. The overexpression of VISTA was confirmed by immunophenotyping of galCOL2 peptide-MHCII-tetramer-enriched antigen-specific CD4+ T cells recovered from BQ mice immunized with COL2/CFA and treated with Aq-galCOL2 (Fig. 10). Of importance, the phenotypic analysis of peptide/MHCII tetramer-enriched galCOL2-specific CD4+ T cells from wt *BQ* mice immunized with COL2 and vaccinated with A^{q}-galCOL2 revealed an identical regulatory T-cell phenotype to the one found in *BQ.HCQ.3tg* mice (Fig. 13).

Moreover, treatment with anti-VISTA neutralizing antibodies reduced the suppressive potency of tolerized galCOL2-specific HCQ3tg T cells in the *ex vivo* suppression assays, indicating a clear impact by VISTA on the therapeutic effect of A^{q}-galCOL2. In contrast, blocking antibodies against CD73 or PD1 showed minimal or no effect, respectively (Fig. 14). The data demonstrate that treatment with A^{q}-galCOL2 generates antigen-specific regulatory T cells. This was observed in both the wildtype (D266) and MMC (266E) models where T cells in the latter had a tolerized phenotype even prior to immunization of the mice with COL2 or treatment with Aq-galCOL2.

### Example 4: Treatment with Aq-galCOL2 expands antigen-specific CD4+ regulatory T cells.

The potential of the human DR4-galCOL2 construct to induce an upregulation of the immune inhibitory receptor VISTA on the CD4+ T cell subsets of peripheral blood monocytes (PBMCs) from RA-patients carrying the DRB1^{∗}0401 allele was investigated. The aim of these studies was the question whether VISTA could mirror the pharmacodynamic effect of DR4-galCol2 on the differentiation of a responsive T cell subset of PBMCs into a tolerogenic phenotype. The blood samples for the study were derived from RA patients fulfilling the respective 1987 revised classification criteria for RA of the American College of Rheumatology and had given their prior written consent to the analyses as part of a study that had obtained ethical approval by the institutional review board of the University Hospital Frankfurt. The study patients had been positively genotyped for DRB1^{∗}0401 carrier status.

As shown in Figure 15 it was demonstrated that an upregulation of VISTA in a subset of CD4+ lymphocytes upon 24 h stimulation with the DR4-glCOL2 construct at a concentration of 5 µg/ml for 24h by measurement of a lymphocyte fraction staining double-positive for a FITC labelled anti-CD4 mAB as well as an BV650-conjugated VISTA-specific mAB by florescence activated flow cytometry (FACS). A detectable increase in the percentage of a VISTA positive CD4+ T cell subset was detectable in a pilot study in two samples and was specifically induced by stimulation with the DR4-galCOL2 construct but not with the anti-CD3/anti-CD28 antibody combination which is the standard stimulus of T cell activation or under culturing conditions in stimulus- free medium for 24h serving for control. However, in initial investigations limited to 4 samples also detected a lack of response to DR4-galCOL2 challenge of PBMCs with respect to VISTA upregulation on CD4+ T lymphocytes as exemplified by the results of the analysis of sample P141 depicted in Figure 15.

In summary the studies show that VISTA is a marker that is upregulated on the cell surface of CD4 + T cell subsets in response to the challenge with MHCII-galCOL2 constructs in mice (*in vivo*) and humans (in *vitro*). Thus, in the mouse the functional role of VISTA on the tolerogenic phenotype induced by the respective murine variant of the therapeutic vaccine A^{q}-galCOL2 could be demonstrated. Since the transfer of a regulatory T cell subset from A^{q}-galCOL2 vaccinated donor mice to naive recipients protects the latter from arthritis induction, the data suggest a major contribution of VISTA to the vaccination effect. The concomitantly performed studies on human PBMCs reveal the capability of human DR4-galCOL2, the homologous variant of A^{q}-galCOL2, to specifically upregulate VISTA on CD4+ T cell subsets of RA patients in a target population of its intended future therapeutic application. The results show that the responses are stimulus-specific, however vary between individual patients. Accordingly, the expression of VISTA on CD4+ T lymphocytes is a marker for a tolerogenic T cell phenotype. It is thus used in a pretreatment in vitro assay to test the individual sensitivity of patient's PBMCs to a variety of immunomodulatory drugs that aim at the induction of regulatory T cells and could improve individualized prediction of treatment response and treatment stratification. Moreover, VISTA could also be used as an *ex vivo* marker to quantify the size of the "tolerogenic" CD4+ subset in an immune monitoring in the long-term follow-up of patients before and after treatment initiation to support therapeutic decisions e.g. timing of retreatment or need to change therapy.

## Claims

1. **A method for the evaluation of a treatment of an autoimmune disorder** in a subject who receives or received the treatment, the method comprising the steps of
**(a)** Providing a biological test sample comprising T-cells of the subject;
**(b)** Determining (i) the level of expression of a V-type immunoglobulin domain-containing suppressor of T-cell activation (VISTA) protein, or VISTA RNA, in the T-cells of the biological test sample; or (ii) determining the number or percentage of VISTA protein and/or VISTA RNA positive T-cells in the biological test sample;
**(c)** Comparing the values as determined in step (b)(i) and/or (b)(ii) with a reference;
preferably wherein reference for (i) is a predetermined level of VISTA protein or VISTA RNA, or wherein the reference for (i) is a level of VISTA protein or VISTA RNA determined in T-cells of a control sample such as a biological sample comprising T-cells of the subject at a time before the treatment or at a time during treatment, but before obtaining the biological test-sample; and/or
preferably wherein the reference for (ii) is a predetermined number/percentage of VISTA protein and/or VISTA RNA positive T-cells, or wherein the reference for (ii) is a number/percentage of VISTA protein and/or VISTA RNA positive T-cells of a control sample such as a biological control sample comprising T-cells of the subject obtained at a time before the treatment or obtained at a time during treatment, but at an earlier time than the biological test sample was obtained;
wherein an increased level in (i) and/or an increased number/percentage in (ii) compared to the respective reference indicates treatment success.

2. **A method for the evaluation of whether a subject is susceptible to a treatment of an autoimmune disorder** in a subject who suffers from the autoimmune disorder and has not yet received the treatment, the method comprising the steps of
**(a)** Providing a biological test sample comprising T-cells of the subject;
**(b)** Determining (i) the level of expression of a V-type immunoglobulin domain-containing suppressor of T-cell activation (VISTA) protein, or VISTA RNA, in the T-cells of the biological test sample; or (ii) determining the number or percentage of VISTA protein and/or VISTA RNA positive T-cells in the biological test sample;
**(c)** Comparing the values as determined in step (b)(i) and/or (b)(ii) with a reference;
preferably wherein reference for (i) is a predetermined level of VISTA protein or VISTA RNA, or wherein the reference for (i) is a level of VISTA protein or VISTA RNA determined in T-cells of a control sample such as a biological sample comprising T-cells of the subject at a time before the treatment or at a time during treatment, but before obtaining the biological test-sample; and/or
preferably wherein the reference for (ii) is a predetermined number/percentage of VISTA protein and/or VISTA RNA positive T-cells, or wherein the reference for (ii) is a number/percentage of VISTA protein and/or VISTA RNA positive T-cells of a control sample such as a biological control sample comprising T-cells of the subject obtained at a time before the treatment or obtained at a time during treatment, but at an earlier time than the biological test sample was obtained;
wherein an increased level in (i) and/or an increased number/percentage in (ii) compared to the respective reference indicates that the subject is susceptible to the treatment.

3. The method of claim 1 or 2, wherein the treatment is the administration of an immune-regulatory agent to the subject, preferably wherein the agent negatively affects a T-cell mediated immune response in the subject.

4. The method of claim 3, wherein the agent is a protein or protein complex binding to a T cell receptor suspected to be expressed in a T-cell of the subject, such as wherein the agent is a protein complex comprising a complex of an MHC class II protein, or a antigenic peptide binding derivative or fragment thereof, and an antigenic peptide comprising an epitope specifically mediating a binding to a T-cell receptor (or a paratope thereon).

5. The method of any one of the preceding claims, wherein the autoimmune disease is mediated by T-cells of the subject.

6. The method of any one of claims 1 to 5, wherein the treatment comprises enhancement of the function or activity of a T regulatory cell, for example by the administration of a peptide-MHC protein complex specifically binding a T-cell receptor expressed on naive T cells or T-cells regulating, driving, or mediating an autoimmune disease.

7. The method of any one of the preceding claims, wherein the biological test sample is selected from whole blood, plasma, serum, synovial fluid, saliva, urine, stool, tears, hair, skin, any other bodily fluid, tissue samples (e.g., biopsy), and cellular extracts thereof.

8. The method of claim 7, wherein the biological test sample is a synovial fluid sample.

9. The method of any one of claims 1 to 8, wherein VISTA is a protein having the amino acid sequence shown in any of SEQ ID NO: 1 to 3, or is a homolog, ortholog, or paralog of the VISTA protein shown in SEQ ID NO: 1.

10. The method of any one of the preceding claims, wherein the level or expression of VISTA protein is determined immunological, for example using a VISTA specific antibody, which may either be labelled fluorescent or is detectable by a fluorescently labelled secondary antibody; or wherein the expression level of the VISTA RNA is detected using Polymerase Chain Reaction (PCR) based methods such as Reverse Transcribed (RT) PCR,, more preferably quantitative RT-PCR (qRT-PCR), or RNA sequencing.

11. The method of claim 10, wherein the VISTA protein is determined immunological, using immunostaining of cell- or tissue samples, Western blotting, ELISA or a fluorescence-activated cell scanning (FACS).

12. The method of any one of the preceding claims, wherein the method involves testing one further biomarkers, preferably wherein the one or more biological markers are selected from CTLA-4, PD-1, Lag-3, Tim-3,TIGIT, FOXP3, FR4 (folate receptor 4) and CD49b (a2 integrin chain), CD73 (ecto-5'-nucleotidase) and interleukin-10.

13. **A compound or composition for use in the treatment of an autoimmune disorder** in a subject, wherein the compound and composition is immune-regulatory and negatively affects a T-cell mediated immune response in the subject when administered, and wherein the treatment further comprises obtaining a biological test sample of the subject at least once before, during and/or subsequent to the administration of the compound or composition, and wherein the subject is stratified as being successfully treated or being susceptible to the treatment by a method according to any one of claim 1 to 30 using the biological test sample of the subject.

14. The compound or composition of claim 13, wherein if an increased level in (i) and/or an increased number/percentage in (ii) compared to the respective reference indicates that the subject is further administered with the compound or composition.

15. The compound or composition of claims 13, wherein if a decreased level in (i) and/or a decreased number/percentage in (ii) compared to the respective reference indicates that the subject is further administered with the compound or composition
